(19) Europäisches Patentamt
European Patent Office
Office européen des brevets

(11) **EP 4 452 848 B1**

(12) **EUROPEAN PATENT SPECIFICATION**

(45) Date of publication and mention
of the grant of the patent:
**08.10.2025   Bulletin 2025/41**

(21) Application number: **22834913.0**

(22) Date of filing: **08.12.2022**

(51) International Patent Classification (IPC):
*C01B 33/12* (2006.01)    *C01B 33/193* (2006.01)
*C01B 33/18* (2006.01)    *C09C 1/30* (2006.01)
*C01B 33/142* (2006.01)    *A61Q 11/00* (2006.01)
*A61K 8/49* (2006.01)    *A61K 8/25* (2006.01)
*A61K 8/02* (2006.01)

(52) Cooperative Patent Classification (CPC):
**C01B 33/12; A61K 8/0241; A61K 8/25;**
**A61Q 11/00; C01B 33/18; C01B 33/193;**
**C09C 1/30;** A61K 2800/412; C01P 2004/62;
C01P 2006/11; C01P 2006/12; C01P 2006/16;
C01P 2006/19

(86) International application number:
**PCT/EP2022/084901**

(87) International publication number:
**WO 2023/117446 (29.06.2023 Gazette 2023/26)**

(54) **PRECIPITATED SILICA AND METHODS THEREOF**

GEFÄLLTES SILIZIUMDIOXID UND VERFAHREN DAFÜR

SILICE PRÉCIPITÉE ET PROCÉDÉS ASSOCIÉS

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **20.12.2021   US 202163291590 P**
**08.03.2022   EP 22160705**

(43) Date of publication of application:
**30.10.2024   Bulletin 2024/44**

(73) Proprietor: **Evonik Operations GmbH**
**45128 Essen (DE)**

(72) Inventors:
• **GALLIS, Karl W.**
**Perryville, Maryland 21903 (US)**
• **SINCLAIR, Fitzgerald A.**
**Smyrna, Delaware 19977 (US)**
• **NASSIVERA, Terry W.**
**Gambrills, Maryland 21054 (US)**
• **DARSILLO, Michael S.**
**Landenberg, Pennsylvania 19350 (US)**
• **LUNDQUIST, Eric G.**
**Essex, MD 21221 (US)**

(74) Representative: **Evonik Patent Association**
**c/o Evonik Industries AG**
**IP Management**
**Postcode 84/339**
**Rodenbacher Chaussee 4**
**63457 Hanau (DE)**

(56) References cited:
EP-A1- 3 216 442        JP-A- H04 288 353
US-A1- 2004 161 389     US-A1- 2012 216 719
US-A1- 2018 168 958

**EP 4 452 848 B1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

The present disclosure relates generally to precipitated silica, and methods of making and using the same.

### BACKGROUND

Porous precipitated silicas are typically produced by the reaction of an alkaline silicate solution, e.g., sodium silicate, with a mineral acid. Commercially, sulfuric acid is mainly used, although other acids such as hydrochloric acid can be applied as well. The acid and the sodium silicate solution are added simultaneously with agitation to water. Precipitated silica arises when silica is precipitated from this dispersion by the neutralization reaction and the generation of by-product sodium salt (sodium sulfate). The precipitated silica consists of aggregates (secondary particles) of primary (or ultimate) colloidal silica particles. The primary particles are mostly spherical and usually have a diameter in the range between 5 and 50 nm. The primary particles in the aggregates are covalently bonded to one another by the formation of siloxane bonds. The aggregates are three-dimensional clusters of these primary particles. The aggregates have diameters of up to 500 nm. The aggregates are not chemically linked into a massive gel network during the preparation process. The aggregates themselves can be physically linked to larger agglomerates of up to 100 $\mu$m in diameter by the formation of hydrogen bonds between the silanol groups on their surfaces before milling). The median agglomerate size is about 20-50 $\mu$m in diameter (before milling). The porosity and surface area of these precipitated silica particles are a function of the size of the primary particles and how they aggregate and agglomerate. The pores are formed by the spaces between the primary particles, and the aggregates. Typical surface areas of commercial precipitated silicas are 5-800 $m^2$/g. They are sold as powders. The tamped density, which is a measure of the weight of these porous powders, is in the range of 50-500 kg/$m^3$. They have a high absorptive capacity of about 175-320 g/100 g.

U.S. Pat. No. 8,597,425 reports that porous precipitated silicas with a primary particle size of 10-80 nm are useful in applications including rubber and tire, a battery separator, an antiblocking agent, a matting agent for inks and paints, a carrier for agricultural products and for feeds, a coating material, a printing ink, a fire-extinguisher powder, a plastic, in the non-impact printing sector, a paper pulp, or an article in the personal care sector,

It is reported in J. Soc Cosmet. Chem August 1978, 29, 497-521 that precipitated silicas with a primary particle size of 12-51 nm are useful in cosmetic applications including toothpaste.

A commercial product SIPERNAT 22 useful in food and feed applications, as a carrier and anticaking- free flow additive is reported to have a primary particle size of 18 nm (Degussa literature No. 64, Physiological Behavior of highly dispersed Oxides of Silicon, Aluminum and Titanium 1978, page 26-27).

U.S. Pat. No. 6,946,119 discloses a precipitated silica product comprising silica particulate comprising silica particles having a median diameter of 1-100 micrometers that support surface deposits thereon comprising an active precipitated amorphous silica a material present in an amount effective to provide a BET specific surface area from 1-50 $m^2$/g for the silica particulate. The precipitated silicas are used in oral care applications.

U.S. Pat. No. 7,255,852 describes a precipitated silica comprising silica product particles having a porous surface, the silica particles having a cumulative surface area for all pores having diameters greater than 500A of less than 8 $m^2$/g, as measured by mercury intrusion, a BET specific surface area of less than approximately 20 $m^2$/g, and a percentage of cetylpyridinium chloride (%CPC) compatibility of greater than 55%. The precipitated silicas are used in oral care applications.

U.S. Pat. No. 7,438,895 discloses an abrasive precipitated silica material with a coating of precipitated silica thereon, wherein said coating of precipitated silica is denser than the material to which it is applied, and wherein said coated precipitated silica material exhibits a median particle size of between 5.5 and 8 microns, a pore area for pores with a diameter greater than 500 A of at most about 2.4 $m^2$/g, and a percentage of cetylpyridinium chloride compatibility after aging said material for 7 days at 140° F. of at least 90%.

U.S 20080160053 describes a method of manufacturing an abrasive silica material, wherein said method involves the following sequential steps: reacting, under high shear mixing conditions, a first amount of silicate and a first amount of acid together, optionally in the presence of at least one electrolyte present in an amount of 5 to 25% weight to weight basis in comparison with the dry weight of the first amount of said silicate, to form a first silica material; and reacting, in the presence of said first silica material, a second amount of silicate and a second amount of acid together, optionally in the presence of at least one electrolyte present in an amount of 5 to 25% weight to weight basis in comparison with the dry weight of the second amount of said silicate, to form a dense phase coating on the surface of said first silica material, thereby forming a silica-coated silica material; wherein said at least one electrolyte is present in either of said steps "a" or "b" or during both steps, and wherein said step "b" is optionally performed under high shear mixing conditions.

U.S. Pat. No. 10,328,002 discloses a dentifrice composition comprising: an abrasive comprising precipitated silica particles characterized by; a BET surface area in a range from about 0.1 to about 9 $m^2$/g; a pack density in a range from about 35 to about 55 lb/ft$^3$ ; an Einlehner abrasion value in a range from about 8 to about 25 mg lost/100,000 revolutions; a total mercury intrusion pore volume in a range from about 0.4-1.2 cc/g; and a stannous compatibility in a range from about 70 to about 99%; wherein the abrasive comprises large pores of a size of approximately 1000 Angstroms or greater and lacks small pores sized less than approximately 500-1000 Angstroms.

WO 2018114280 describes silica particles with: a BET surface area in a range from about 0.1 to about 7 m$^2$ /g; a pack density in a range from about 35 to about 55 lb/ft$^3$; an Einlehner abrasion value in a range from about 8 to about 25 mg lost/100,000 revolutions; a total mercury intrusion pore volume in a range from about 0.7 to about 1.2 cc/g; and (v) a stannous compatibility in a range from about 70 to about 99%.

U.S. 20190374448 discloses a dentifrice composition comprising: binder; surfactant; silica particles; wherein the silica particles comprise: a d50 median particle size in a range from about 4 to about 25 $\mu$m; a BET surface area in a range from 0 to about 10 m$^2$/g; and a total mercury intrusion pore volume in a range from about 0.2 to about 1.5 cc/g.

WO 2019238777 describes silica particles characterized by: (i) d50 median particle size in a range from about 8 to about 20 $\mu$m; (ii) a sphericity factor (S80) of greater than or equal to about 0.9; (iii) a BET surface area in a range from about 0.1 to about 8 m$^2$/g; (iv) a total mercury intrusion pore volume in a range from about 0.35 to about 0.8 cc/g; and (v) a loss on ignition (LOI) in a range from about 3 to about 7 wt.

US 2012/216719 A1 discloses a precipitated silica having a BET surface area of 33m2/g, an oil absorption of 62 cc/100g and a mercury intruded volume of 0.95 ml/g. The document does not provide any indication on the primary particle size.

The ability of precipitated silica to provide compatibility with ingredients while providing the correct balance of cleaning and abrasion is important in a toothpaste formulation. None of the prior art addresses the problem of lack of compatibility with other ingredients, like CPC and BAC, and flavor compatibility and to achieve PCR (80-110) and RDA (100-220) values in the normal range at the same time.

SUMMARY

The inventors of the present invention have now found that silicas with high primary particle size can lead to high compatibility with CPC, BAC and/or flavor in oral care applications with acceptable PCR/RDA values in the range of current silica cleaning agents.

Subject of the invention is therefore precipitated silicas that are characterized by a primary particle size mean of greater than 80 nm, preferably greater than 90 nm, more preferably greater than 100 nm, still preferably greater than 110 nm, most preferably between 120 nm and 500 nm, a BET surface area of 10-40 m$^2$/g, preferably 10-26 m$^2$/g, more preferably 10-23 m$^2$/g, most preferably 10-20 m$^2$/g, a total mercury intruded volume of 0.75-2.00 cc/g, preferably 0.80-1.80 cc/g, more preferably 0.85-1.65 cc/g, more preferably 0.90-1.50 cc/g and an oil absorption of 60-120 cc/100g, preferably 60-110 cc/100g more preferably 60-100 cc/100g, most preferably 60-90 cc/100g.

Subject of the invention is also a process comprising

(a) continuously feeding an acid and an alkali metal silicate or alkaline earth metal silicate into a liquid medium at a silicate addition rate V1 and a temperature 70-96°C with stirring to form the silica particles,
(b) stopping the feed of alkali metal silicate or alkaline earth metal silicate and acid and then increasing the temperature to 90 to 100°C, preferably 94 to 96 °C, with stirring,
(c) adding an alkali metal silicate or alkaline earth metal silicate and an acid with stirring, wherein the silicate addition rate is 1 to 40 %, preferably 1 to 30 %, more preferably 2 to 10 %, still preferably 3 to 5 %, of the silicate addition rate V1 and the pH value of 9.0 to 10.0, preferably 9.5 to 9.9, more preferably 9.6 to 9.8, is kept constant during the addition of the alkali metal silicate or alkaline earth metal silicate, by adjusting the acid rate,
(d) stopping the addition of alkali metal silicate or alkaline earth metal silicate and adding acid with stirring until the pH of 5.0 to 7.0, preferably 5.5 to 6.5, is reached.

A further subject of the invention is the use of the inventive silicas in cosmetics, anti-caking free/flow, food, carrier applications, dentifrice and mouthwash.

DETAILED DESCRIPTION OF THE INVENTION

The inventive precipitated silicas have a primary particle size mean of greater than 80 nm, preferably greater than 90 nm, more preferably greater than 100 nm, still preferably greater than 110 nm, most preferably between 120 nm and 500 nm, a BET surface area of 10-40 m$^2$/g, preferably 10-26 m$^2$/g, more preferably 10-23 m$^2$/g, most preferably 10-20 m$^2$/g, a total mercury intruded volume of 0.75-2.00 cc/g, preferably 0.80-1.80 cc/g, more preferably 0.85-1.65 cc/g, more preferably 0.90-1.50 cc/g and an oil absorption of 60-120 cc/100g, preferably 60-110 cc/100g more preferably 60-100 cc/100g, most preferably 60-90 cc/100g.

The silica of the invention has a high primary particle size.

The precipitated silica according to the invention could have a CTAB surface area of 10-35 m$^2$/g, preferably 20-30 m$^2$/g.

The precipitated silica according to the invention could have a pack density of 0.40-0.80 g/cm$^3$, preferably 0.48-0.75 g/cm$^3$.

The precipitated silica according to the invention could have an Einlehner value of less than 18 mg lost/100k revolutions, preferably 3 to 18 mg lost/100k revolutions.

The C-content of the silica according to the invention can be lower than 3%, preferably lower than 1%, more preferably 0% to 0.5%, most preferably 0% to 0.1%.

The precipitated silica according to the invention could have a primary particle size mean of greater than 90 nm, a BET surface area of 10-26 m$^2$/g, a total mercury intruded volume of 0.75-2.00 cc/g and an oil absorption of 60-120 cc/100g.

The precipitated silica according to the invention could have a primary particle size mean of greater than 80 nm, a BET surface area of 10-30 m$^2$/g, a total mercury intruded volume of 0.80-1.80 cc/g and an oil absorption of 70-110 cc/100g.

The precipitated silica according to the invention could have a primary particle size mean of greater than 90 nm, a BET surface area of 10-26 m$^2$/g, a total mercury intruded volume of 0.80-1.80 cc/g and an oil absorption of 70-110 cc/100g.

The precipitated silica according to the invention could have a primary particle size mean of greater than 100 nm, a BET surface area of 10-23 m$^2$/g, a total mercury intruded volume of 0.75-2.00 cc/g and an oil absorption of 60-120 cc/100g.

The precipitated silica according to the invention could have a primary particle size mean of greater than 80 nm, a BET surface area of 10-30 m$^2$/g, a total mercury intruded volume of 0.85-1.65 cc/g and an oil absorption of 60-100 cc/100g.

The precipitated silica according to the invention could have a primary particle size mean of greater than 100 nm, a BET surface area of 10-23 m$^2$/g, a total mercury intruded volume of 0.85-1.65 cc/g and an oil absorption of 60-100 cc/100g.

The precipitated silica according to the invention could have a primary particle size mean of 120-500 nm, a BET surface area of 10-20 m$^2$/g, a total mercury intruded volume of 0.75-2.00 cc/g and an oil absorption of 60-120 cc/100g.

The precipitated silica according to the invention could have a primary particle size mean of greater than 80 nm, a BET surface area of 10-30 m$^2$/g, a total mercury intruded volume of 0.90-1.50 cc/g and an oil absorption of 60-90 cc/100g.

The precipitated silica according to the invention could have a primary particle size mean of 120-500 nm, a BET surface area of 10-20 m$^2$/g, a total mercury intruded volume of 0.90-1.50 cc/g and an oil absorption of 60-90 cc/100g.

The process according to the invention comprises at least four steps:

(a) continuously feeding an acid and an alkali metal silicate or alkaline earth metal silicate into a liquid medium at a silicate addition rate V1 and a temperature of 70-96°C with stirring to form the silica particles.

(b) stopping the feed of alkali metal silicate or alkaline earth metal silicate and acid and then increasing the temperature to 90 to 100°C, preferably 94 to 96 °C, with stirring,

(c) adding an alkali metal silicate or alkaline earth metal silicate and an acid with stirring, wherein the silicate addition rate is 1 to 40 %, preferably 5 to 30 %, more preferably 7 to 25 %, still preferably 10 to 20 %, of the silicate addition rate V1 and the pH value of 9.0 to 10.0, preferably 9.5 to 9.9, more preferably 9.6 to 9.8, is kept constant during the addition of the alkali metal silicate or alkaline earth metal silicate, by adjusting the acid rate,

(d) stopping the addition of alkali metal silicate or alkaline earth metal silicate and adding acid with stirring until the pH of 5.0 to 7.0, preferably 5.5 to 6.5 is reached.

The silica of step (d) could be filtered (e), e.g. in a filter press.

The silica of step (e) could be dried, e.g. in a spray dryer (f).

The silica of step (f) could be milled,

Preferably 35-65% of the total volume of alkali metal silicate or alkaline earth metal silicate is added during step (a).

The liquid medium in step (a) could be alkali metal silicate or alkaline earth metal silicate and water.

The temperature range in step (a) could be 70 to 95 °C, preferably 70 to 90°C, more preferably 80 to 90°C.

The acid rate in the step (a) could be sufficient to maintain a pH between 8.5-10.5, more preferably 9.5-10.2.

The silicate rate in step (c) could be slowed to 5-30% of the rate in step (a), more preferably 10-20%.

The alkali metal silicate in step (a) and (c) could be preferably sodium silicate.

The silicate addition rate in step (c) could be adjusted so that the % of silicate added/hr in this step is less 30 %/hr, preferably 15-25 %/hr, based on the total amount of silicate added during the batch. The % silicate added/hr is calculated by: volume of silicate used in step (c) / time in hours of step (c) / volume of silicate used in step (a) and step (c) *100.

The acid in step (a), (c) and (d) could be preferably sulfuric acid.

The period of time for step (c) could be 100 to 500 minutes, preferably 150 to 300 minutes.

The inventive precipitated silica can be produced by the inventive process.

The inventive precipitated silica could be used in cosmetics, anti-caking free/flow, food, carrier applications, dentifrice and mouthwash.

The inventive precipitated silicas have an improved compatibility with cetylpyridinium chloride (CPC), benzalkonium chloride (BAC) and flavor in oral care applications.

Examples

Primary Particle Size Mean by SEM

Images were taken by scanning electron microscopy at a magnification of 50,000 times. The images were sputtered with platinum and care was taken so the sputtering did not cause texturing of the particles surface, as this could be mistaken for primary structure. The image must be representative of the entire sample and contain a minimum of 30 particles. The primary particles were then measured. If the particles were not completely round, the smallest diameter across each particle was used. Particles that are on the edges of the image that cannot be completely observed should not be used. Mean and median values were then calculated based on the data set.

Carbon content

Carbon content was measured on a LECO SC832 Carbon/Sulfur analyzer.

Brightness

Silica samples were pressed into a smooth surfaced pellet and analyzed using a Technidyne Brightmeter S-5/BC. This instrument has a dual beam optical system where the sample is illuminated at an angle of 45°, and the reflected light is viewed at 0°. It conforms to TAPPI test methods T452 and T646, and ASTM Standard D985. Powdered materials are pressed to about a 1 cm pellet with enough pressure to give a pellet surface that is smooth and without loose particles or gloss.

Moisture

The moisture was determined by heating the silica at 105 °C for 2 hours.

BET Surface Area

The BET surface areas of silicas of the invention were determined with a Micromeritics TriStar 3020 instrument by the BET nitrogen adsorption method of Brunaur et al., J. Am. Chem. Soc., 60, 309 (1938), which is known in the field of particulate materials, such as silica and silicate materials. Oil Absorption

Oil absorption values were determined in accordance with the rub-out method described in ASTM D281 using linseed oil (cc oil absorbed per 100 g of the particles). Generally, a higher oil absorption level indicates a higher structure particle, while a lower value typically indicates a lower structure particle.

Total Mercury intrusion volume

Mercury intruded volume or total pore volume (Hg) was measured by mercury porosimetry using a Micromeritics AutoPore IV 9520 (or, Micromeritics AutoPore V 9620) apparatus. The pore diameters was calculated by the Washburn equation employing a contact angle Theta ($\Theta$) equal to 130° and a surface tension gamma equal to 484 dynes/cm. Mercury was forced into the voids of the particles as a function of pressure and the volume of the mercury intruded per gram of sample was calculated at each pressure setting. Total pore volume expressed herein represents the cumulative volume of mercury intruded at pressures from vacuum to 60,000 psi. Increments in volume ($cm^3/g$) at each pressure setting were plotted against the pore radius or diameter corresponding to the pressure setting increments. The peak in the intruded volume versus pore radius or diameter curve corresponds to the mode in the pore size distribution and identifies the most common pore size in the sample. Specifically, sample size was adjusted to achieve a stem volume of 25-90% in a powder penetrometer with a 5 mL bulb and a stem volume of about 1.1 mL. Samples were evacuated to a pressure of 50 $\mu$m of Hg and held for 5 minutes. Mercury filled the pores from 4.0 to 60,000 psi with a 10 second equilibrium time at each data collection point). The total pore volume as described above captures the volumes from intraparticle porosity resulting from the pore structure within the individual particles, as well as, the interparticle porosity formed from the interstitial spacing of the packed particles under pressure. To better separate and measure examining the actual intraparticle porosity of the produced amorphous silicas, the pore volume of pores <0.11 $\mu$m can be used.

Einlehner

Einlehner AT-1000 Abrader is used as follows: (1) a Fourdrinier brass wire screen that has been previously cleaned and dried was then weighed and exposed to the action of a 10% aqueous silica suspension for a fixed length of time, specifically 100g of sample in 900g of deionized water; (2) the amount of abrasion was then determined as milligrams brass lost from the Fourdrinier wire screen per 100,000 revolutions. The result, measured in units of mg loss, was characterized as the 10% brass Einlehner (BE) abrasion value.

CTAB Surface Area

The CTAB surface areas disclosed herein were determined by absorption of CTAB (cetyltrimethylammonium bromide) on the silica surface, the excess separated by centrifugation and the quantity determined by titration with sodium lauryl sulfate using a surfactant electrode. Specifically, about 0.5 grams of the silica particles were placed in a 250-mL beaker with 100 mL CTAB solution (5.5 g/L), mixed on an electric stir plate for 1 hour, then centrifuged for 30 min at 10,000 RPM. One mL of 10% Triton X-100 was added to 5 mL of the clear supernatant in a 100-mL beaker. The pH was adjusted to 3-3.5 with 0.1 N HCl and the specimen was titrated with 0.01 M sodium lauryl sulfate using a surfactant electrode (Brinkmann SUR1501-DL) to determine the endpoint.

Particle Size

Measurement of the particle size of the silicas of the invention was conducted on HORIBA Laser Scattering Dry Particle Size Distribution Analyzer LA-960 through the angle of scattered laser light.

Water Corrected AbC Value

Water absorption values were determined with an Absorptometer "C" torque rheometer from C.W. Brabender Instruments, Inc. Approximately 1/3 of a cup of the silica sample was transferred to the mixing chamber of the Absorptometer and mixed at 150 RPM. Water was then added at a rate of 6 mL/min, and the torque required to mix the powder was recorded. As water was absorbed by the powder, the torque reached a maximum as the powder transformed from free-flowing to a paste. The total volume of water added when the maximum torque was reached is then standardized to the quantity of water that could be absorbed by 100 g of powder. Since the powder was used on an as received basis (not previously dried), the free moisture value of the powder was used to calculate a "moisture corrected water AbC value" by the following equation.

$$Water\ Corrected\ AbC\ Value = \frac{water absorbed(cc) + \%moisture}{(100(g) - \%moisture)/100}$$

5 wt. % pH

5% pH was measured by weighing 5.0 g of sample out to the nearest 0.1g and transferring the weighed sample to a 250mL beaker. 95mL of DI water was added and the sample was stirred for 5min. The pH was then measured with the pH meter while the sample was being stirred.

Pack Density and Pour Density

Pack density and pour density were measured by placing 20.0g of the sample into a 250 mL graduated cylinder with a flat rubber bottom. The initial volume was recorded and used to calculate the pour density by dividing it into the weight of sample used. The cylinder was then placed onto a tap density machine where it was rotated on a cam at a specific RPM. The cam was designed to raise and drop the cylinder a distance of 5.715 cm once per second, until the sample volume was constant, typically for 15 min. This final volume was recorded and used to calculate the packed density by dividing it into the weight of sample used.

Example 1 A: (Inventive Example)

383 mL of sodium silicate (2.65 MR, 1.193g/mL) and 957 mL of water were added to a 7 L laboratory sized reactor and heated 85 °C with stirring from an overhead stirrer equipped with a propeller blade at 350 RPM. Once 85 °C was reached, sodium silicate (2.65 MR, 1.193g/ml) and sulfuric acid (1.121g/mL) were added simultaneously for 38 minutes at rates of 39.0 mL/min and 16.4 mL/min, respectively. After 38 minutes elapsed, the flow of sodium silicate and sulfuric acid were stopped, and the reaction mixture was heated to 95 °C. Once 95 °C was reached, sodium silicate (2.65 MR, 1.193g/mL) was added at 10.2 mL/min and sulfuric acid (1.121 g/ml) was added at a rate sufficient to maintain pH 9.6 (+/- 0.1), for an additional 150 minutes. After the additional 150 minutes elapsed, the flow of sodium silicate was stopped, and the pH was adjusted to pH 6.0 with continued flow of sulfuric acid (1.121 g/mL) at a rate of 5.1 mL/min. Once the pH was stabilized at pH 6.0, the batch was filtered, washed with 14L of water and was dried overnight at 105 °C.

Example 1 B: (Inventive Example)

273 mL of sodium silicate (2.65 MR, 1.193g/mL) and 681 mL of water were added to a 7 L laboratory sized reactor and heated 85 °C with stirring from an overhead stirrer equipped with a propeller blade at 350 RPM. Once 85 °C was reached, sodium silicate (2.65 MR, 1.193g/ml) and sulfuric acid (1.121g/mL) were added simultaneously for 38 minutes at rates of 27.7 mL/min and 11.7 mL/min, respectively. After 38 minutes elapsed, the flow of sodium silicate and sulfuric acid were stopped, and the reaction mixture was heated to 95 °C. Once 95 °C was reached, sodium silicate (2.65 MR, 1.193g/mL) was added at 10.7 mL/min and sulfuric acid (1.121 g/ml) was added at a rate sufficient to maintain pH 9.6 (+/- 0.1), for an additional 210 minutes. After the additional 210 minutes elapsed, the flow of sodium silicate was stopped, and the pH was adjusted to pH 6.0 with continued flow of sulfuric acid (1.121 g/mL) at a rate of 5.1 mL/min. Once the pH was stabilized at pH 6.0, the batch was filtered, washed with 14L of water and was dried overnight at 105 °C.

Example 1 C: (Inventive Example)

273 mL of sodium silicate (2.65 MR, 1.193g/mL) and 681 mL of water were added to a 7 L laboratory sized reactor and heated 85 °C with stirring from an overhead stirrer equipped with a propeller blade at 350 RPM. Once 85 °C was reached, sodium silicate (2.65 MR, 1.193g/ml) and sulfuric acid (1.121g/mL) were added simultaneously for 38 minutes at rates of 43.8 mL/min and 18.4 mL/min, respectively. After 38 minutes elapsed, the flow of sodium silicate and sulfuric acid were stopped, and the reaction mixture was heated to 95 °C. Once 95 °C was reached, sodium silicate (2.65 MR, 1.193g/mL) was added at 9.8 mL/min and sulfuric acid (1.121 g/ml) was added at a rate sufficient to maintain pH 9.6 (+/- 0.1), for an additional 120 minutes. After the additional 120 minutes elapsed, the flow of sodium silicate was stopped, and the pH was adjusted to pH 6.0 with continued flow of sulfuric acid (1.121 g/mL) at a rate of 4.7 mL/min. Once the pH was stabilized at pH 6.0, the batch was filtered, washed with 14L of water and was dried overnight at 105 °C.

Example 2A: (Inventive Example)

320 mL of sodium silicate (2.65 MR, 1.193g/mL) and 824 mL of water were added to a 7 L laboratory sized reactor and heated 85 °C with stirring from an overhead stirrer equipped with a propeller blade at 350 RPM. Once 85 °C was reached, sodium silicate (2.65 MR, 1.193g/ml) and sulfuric acid (1.121g/mL) were added simultaneously for 47 minutes at rates of 33.8 mL/min and 15.1 mL/min, respectively. After 47 minutes elapsed, the flow of sodium silicate and sulfuric acid were stopped, and the reaction mixture was heated to 95 °C. Once 95 °C was reached, sodium silicate (2.65 MR, 1.193g/mL) was added at 10.4 mL/min and sulfuric acid (1.121 g/ml) was added at a rate sufficient to maintain pH 9.6 (+/- 0.1), for an additional 150 minutes. After the additional 150 minutes elapsed, the flow of sodium silicate was stopped, and the pH was adjusted to pH 6.0 with continued flow of sulfuric acid (1.121 g/mL) at a rate of 5.0 mL/min. Once the pH was stabilized at pH 6.0, the batch was filtered, washed with 14L of water and was dried overnight at 105 °C.

Table 1 shows the analytical values of the Examples.

Table 1

| Example | Primary Particle Size Mean, SEM (nm) | Primary Particle Size Median, SEM (nm) | Number of particles counted | Total Hg intruded Volume (cc/g) | HgI Pore Volume for pores < 1 $\mu$m (cc/g) | BET SA (m$^2$/g) | CTAB SA (m$^2$/g) |
|---------|------|------|-----|------|------|----|----|
| 1A | 120 | 110 | 51 | 1.68 | 1.29 | 20 | 21 |
| 1B | 145 | 100 | 38 | 0.93 | 0.43 | 11 | 10 |
| 1C | 150 | 115 | 38 | 0.78 | 0.36 | 38 | 14 |
| 2A | 113 | 100 | 50 | 1.35 | 0.74 | 17 | 16 |

Comparative Example 5A:

594 mL of sodium silicate (2.65 MR, 1.193g/mL, heated to 80 °C) and 1483 mL of water were added to a 7 L reactor and heated 85 °C with stirring at 350 RPM. Once 85 °C was reached, sodium silicate (2.65 MR, 1.193g/ml, heated to 80 °C) and sulfuric acid (1.121g/mL) were added simultaneously for 38 minutes at rates of 60.4 mL/min and 25.3 mL/min, respectively. After 38 minutes elapsed, the flow of sodium silicate was stopped, and the pH was adjusted to pH 6.0 with continued flow of sulfuric acid (1.121 g/mL) at a rate of 25.3 mL/min. Once the pH was stabilized at pH 6.0, the batch was filtered, washed with 14 L of water and was oven dried overnight.

Comparative Example 5B:

594 mL of sodium silicate (2.65 MR, 1.193g/mL, heated to 80 °C) and 1483 mL of water were added to a 7 L reactor and heated 85 °C with stirring at 350 RPM. Once 85 °C was reached, sodium silicate (2.65 MR, 1.193g/ml, heated to 80 °C) and sulfuric acid (1.121g/mL) were added simultaneously for 38 minutes at rates of 60.4 mL/min and 21.6 mL/min, respectively. After 38 minutes elapsed, the flow of sodium silicate was stopped, and the pH was adjusted to pH 6.0 with continued flow of sulfuric acid (1.121 g/mL) at a rate of 21.6 mL/min. Once the pH was stabilized at pH 6.0, the batch was filtered, washed with 14 L of water and was oven dried overnight.

Comparative Example 5C:

594 mL of sodium silicate (2.65 MR, 1.193g/mL, heated to 80 °C) and 1483 mL of water were added to a 7 L reactor and heated 85 °C with stirring at 350 RPM. Once 85 °C was reached, sodium silicate (2.65 MR, 1.193g/ml, heated to 80 °C) and sulfuric acid (1.121g/mL) were added simultaneously for 38 minutes at rates of 60.4 mL/min and 29.2 mL/min, respectively. After 38 minutes elapsed, the flow of sodium silicate was stopped, and the pH was adjusted to pH 6.0 with continued flow of sulfuric acid (1.121 g/mL) at a rate of 29.2 mL/min. Once the pH was stabilized at pH 6.0, the batch was filtered, washed with 14 L of water and was oven dried overnight.

Comparative Example 5D:

594 mL of sodium silicate (2.65 MR, 1.193g/mL, heated to 80 °C) and 1483 mL of water were added to a 7 L reactor and heated 85 °C with stirring at 350 RPM. Once 85 °C was reached, sodium silicate (2.65 MR, 1.193g/ml, heated to 80 °C) and sulfuric acid (1.121 g/mL) were added simultaneously for 38 minutes at rates of 60.4 mL/min and 29.2 mL/min, respectively. After 38 minutes elapsed, the flow of sodium silicate was stopped, and the pH was adjusted to pH 6.0 with continued flow of sulfuric acid (1.121 g/mL) at a rate of 29.2 mL/min. Once the pH was stabilized at pH 6.0, the batch was filtered, washed with 14 L of water and was oven dried overnight.

Comparative Example 5E:

594 mL of sodium silicate (2.65 MR, 1.193g/mL, heated to 80 °C) and 1483 mL of water were added to a 7 L reactor and heated 85 °C with stirring at 350 RPM. Once 85 °C was reached, sodium silicate (2.65 MR, 1.193g/ml, heated to 80 °C) and sulfuric acid (1.121g/mL) were added simultaneously for 38 minutes at rates of 60.4 mL/min and 19.1 mL/min, respectively. After 38 minutes elapsed, the flow of sodium silicate was stopped, and the pH was adjusted to pH 6.0 with continued flow of sulfuric acid (1.121 g/mL) at a rate of 19.1 mL/min. Once the pH was stabilized at pH 6.0, the batch was filtered, washed with 14 L of water and was oven dried overnight.

Table 2 shows the analytical values of the Examples-

Table 2

| Example | Opalescence Time (min) | Primary Particle Size Mean, SEM (nm) | Total Hg intruded Volume (cc/g) | BET SA $(m^2/g)$ | CTAB SA $(m^2/g)$ |
|---|---|---|---|---|---|
| 5A | 15 | 20 | 2.87 | 62 | 61 |
| 5B | 25 | 60 | 2.73 | 217 | 47 |
| 5C | 11 | 10 | 2.62 | 58 | 52 |
| 5D | 26 | 50 | 2.96 | 272 | 66 |
| 5E | 33 | 60 | 2.75 | 46 | 55 |

Comparative Example 6A

594 mL of sodium silicate (2.65 MR, 1.193g/mL, heated to 80 °C) and 1483 mL of water were added to a 7 L reactor and heated 80 °C with stirring at 350 RPM. Once 85 °C was reached, sodium silicate (2.65 MR, 1.193g/ml, heated to 80 °C) and sulfuric acid (1.121g/mL) were added simultaneously for 38 minutes at rates of 60.4 mL/min and 21.6 mL/min, respectively. After 38 minutes elapsed, the flow of sodium silicate was stopped, and the pH was adjusted to pH 6.0 with continued flow of sulfuric acid (1.121 g/mL) at a rate of 21.6 mL/min. Once the pH was stabilized at pH 6.0, the batch was filtered, washed with 14 L of water and was oven dried overnight.

Comparative Example 6B

594 mL of sodium silicate (2.65 MR, 1.193g/mL, heated to 85 °C) and 1483 mL of water were added to a 7 L reactor and heated 90 °C with stirring at 350 RPM. Once 85 °C was reached, sodium silicate (2.65 MR, 1.193g/ml, heated to 85 °C) and sulfuric acid (1.121g/mL) were added simultaneously for 38 minutes at rates of 60.4 mL/min and 21.6 mL/min, respectively. After 38 minutes elapsed, the flow of sodium silicate was stopped, and the pH was adjusted to pH 6.0 with continued flow of sulfuric acid (1.121 g/mL) at a rate of 21.6 mL/min. Once the pH was stabilized at pH 6.0, the batch was filtered, washed with 14 L of water and was oven dried overnight.

Comparative Example 6C

594 mL of sodium silicate (2.65 MR, 1.193g/mL, heated to 90 °C) and 1483 mL of water were added to a 7 L reactor and heated 95 °C with stirring at 350 RPM. Once 85 °C was reached, sodium silicate (2.65 MR, 1.193g/ml, heated to 90 °C) and sulfuric acid (1.121g/mL) were added simultaneously for 38 minutes at rates of 60.4 mL/min and 21.6 mL/min, respectively. After 38 minutes elapsed, the flow of sodium silicate was stopped, and the pH was adjusted to pH 6.0 with continued flow of sulfuric acid (1.121 g/mL) at a rate of 21.6 mL/min. Once the pH was stabilized at pH 6.0, the batch was filtered, washed with 14 L of water and was oven dried overnight.

Table 3 shows the analytical values of the Examples

Table 3

| Example | Reaction Temperature (°C) | Opalescence Time (min) | Primary Particle Size Mean, SEM (nm) | Total Hg intruded Volume (cc/g) | BET SA (m²/g) | CTAB SA (m²/g) |
|---|---|---|---|---|---|---|
| 6A | 80 | 24 | 20 | 2.14 | 62 | 47 |
| 6B | 90 | 20 | 25 | 2.96 | 52 | 52 |
| 6C | 95 | 18 | 50 | 2.00 | 39 | 38 |

Comparative Example 7A:

594 mL of sodium silicate (2.65 MR, 1.193g/mL, heated to 80 °C) and 1483 mL of water were added to a 7 L reactor and heated 85 °C with stirring at 650 RPM. Once 85 °C was reached, sodium silicate (2.65 MR, 1.193g/ml, heated to 80 °C) and sulfuric acid (1.121g/mL) were added simultaneously for 38 minutes at rates of 60.4 mL/min and 25.4 mL/min, respectively. After 38 minutes elapsed, the flow of sodium silicate was stopped, and the pH was adjusted to pH 6.0 with continued flow of sulfuric acid (1.121 g/mL) at a rate of 25.4 mL/min. Once the pH was stabilized at pH 6.0, the batch was filtered, washed with 14 L of water and was oven dried overnight.

Table 4 shows the analytical values of the Example.

Table 4

| Example | Primary Particle Size Mean, SEM (nm) | Total Hg intruded Volume (cc/g) | BET SA (m²/g) | CTAB SA (m²/g) |
|---|---|---|---|---|
| 7A | 40 | 2.12 | 29 | 23 |

Comparative Example 8A

510 mL of sodium silicate (2.65 MR, 1.193g/mL, heated to 80 °C) and 1310 mL of water were added to a 7 L reactor and heated 80 °C with stirring at 350 RPM. Once 85 °C was reached, sodium silicate (2.65 MR, 1.193g/ml, heated to 80 °C) and sulfuric acid (1.121g/mL) were added simultaneously for 38 minutes at rates of 53.7 mL/min and 24.0 mL/min, respectively. After 38 minutes elapsed, the flow of sodium silicate was stopped, and the pH was adjusted to pH 6.0 with continued flow of sulfuric acid (1.121 g/mL) at a rate of 24.0 mL/min. Once the pH was stabilized at pH 6.0, the batch was filtered, washed with 14 L of water and was oven dried overnight.

Comparative Example 8B

510 mL of sodium silicate (2.65 MR, 1.193g/mL, heated to 80 °C) and 1310 mL of water were added to a 7 L reactor and heated 80 °C with stirring at 350 RPM. Once 85 °C was reached, sodium silicate (2.65 MR, 1.193g/ml, heated to 80 °C) and sulfuric acid (1.121g/mL) were added simultaneously for 38 minutes at rates of 53.7 mL/min and 27.5 mL/min, respectively. After 38 minutes elapsed, the flow of sodium silicate was stopped, and the pH was adjusted to pH 6.0 with continued flow of sulfuric acid (1.121 g/mL) at a rate of 27.5 mL/min. Once the pH was stabilized at pH 6.0, the batch was filtered, washed with 14 L of water and was oven dried overnight.

Comparative Example 8C

510 mL of sodium silicate (2.65 MR, 1.193g/mL, heated to 80 °C) and 1310 mL of water were added to a 7 L reactor and heated 80 °C with stirring at 350 RPM. Once 85 °C was reached, sodium silicate (2.65 MR, 1.193g/ml, heated to 80 °C) and sulfuric acid (1.121g/mL) were added simultaneously for 38 minutes at rates of 53.7 mL/min and 20.4 mL/min, respectively. After 38 minutes elapsed, the flow of sodium silicate was stopped, and the pH was adjusted to pH 6.0 with continued flow of sulfuric acid (1.121 g/mL) at a rate of 20.4 mL/min. Once the pH was stabilized at pH 6.0, the batch was filtered, washed with 14 L of water and was oven dried overnight.

Comparative Example 8D

510 mL of sodium silicate (2.65 MR, 1.193g/mL, heated to 80 °C) and 1310 mL of water were added to a 7 L reactor and heated 80 °C with stirring at 350 RPM. Once 85 °C was reached, sodium silicate (2.65 MR, 1.193g/ml, heated to 80 °C) and sulfuric acid (1.121g/mL) were added simultaneously for 38 minutes at rates of 53.7 mL/min and 19.2 mL/min, respectively. After 38 minutes elapsed, the flow of sodium silicate was stopped, and the pH was adjusted to pH 6.0 with continued flow of sulfuric acid (1.121 g/mL) at a rate of 19.2 mL/min. Once the pH was stabilized at pH 6.0, the batch was filtered, washed with 14 L of water and was oven dried overnight.

Comparative Example 8E

510 mL of sodium silicate (2.65 MR, 1.193g/mL, heated to 80 °C) and 1310 mL of water were added to a 7 L reactor and heated 80 °C with stirring at 350 RPM. Once 85 °C was reached, sodium silicate (2.65 MR, 1.193g/ml, heated to 80 °C) and sulfuric acid (1.121g/mL) were added simultaneously for 38 minutes at rates of 53.7 mL/min and 18.0 mL/min, respectively. After 38 minutes elapsed, the flow of sodium silicate was stopped, and the pH was adjusted to pH 6.0 with continued flow of sulfuric acid (1.121 g/mL) at a rate of 18.0 mL/min. Once the pH was stabilized at pH 6.0, the batch was filtered, washed with 14 L of water and was oven dried overnight.

Table 5 shows the analytical values of the Examples

Table 5

| Example | Opalescence Time (min) | Primary Particle Size Mean, SEM (nm) | Total Hg intruded Volume (cc/g) | BET SA (m²/g) | CTAB SA (m²/g) |
|---------|------------------------|--------------------------------------|--------------------------------|---------------|----------------|
| 8A | 14 | 40 | 2.45 | 49 | 43 |
| 8B | 11 | 15 | 2.48 | 58 | 51 |
| 8C | 20 | 50 | 2.45 | 52 | 36 |
| 8D | 20 | 40 | 2.63 | 54 | 33 |
| 8E | 25 | 70 | 2.54 | 47 | 35 |

Example 10A: (Inventive Example)

62 L of sodium silicate (2.65 MR, 1.193g/mL, heated to 80 °C) and 154 L of water were added to a 1200 L reactor and heated 85 °C with stirring at 80 RPM and recirculation at 80 L/min. Once 85 °C was reached, sodium silicate (2.65 MR, 1.193g/ml, heated to 80 °C) and sulfuric acid (1.121g/mL) were added simultaneously for 38 minutes at rates of 6.27 L/min and 2.24 L/min, respectively. After 38 minutes elapsed, the flow of sodium silicate and sulfuric acid were stopped, and the reaction mixture was heated to 95 °C. Once 95 °C was reached, sodium silicate (2.65 MR, 1.193g/mL, heated to 80 °C) was added at 1.63 L/min and sulfuric acid (1.121 g/ml) was added at a rate sufficient to maintains pH 9.6 (+/- 0.1), for an additional 150 minutes. After the additional 150 minutes elapsed, the flow of sodium silicate was stopped, and the pH was adjusted to pH 6.0 with continued flow of sulfuric acid (1.121 g/mL) at a rate of 0.82 mL/min. Once the pH was stabilized at pH 6.0, the batch was filtered, washed until a conductivity of <1500 μS, was spray dried to a target moisture of 5% and was milled to a particle size of approximately 10 μm.

Example 10B: (Inventive Example)

53 L of sodium silicate (2.65 MR, 1.193g/mL, heated to 80 °C) and 133 L of water were added to a 1200 L reactor and heated 85 °C with stirring: at 80 RPM and recirculation at 80 L/min. Once 85 °C was reached, sodium silicate (2.65 MR, 1.193g/ml, heated to 80 °C) and sulfuric acid (1.121g/mL) were added simultaneously for 38 minutes at rates of 5.42 L/min and 1.82 L/min, respectively. After 38 minutes elapsed, the flow of sodium silicate and sulfuric acid were stopped, and the reaction mixture was heated to 95 °C. Once 95 °C was reached, sodium silicate (2.65 MR, 1.193g/mL, heated to 80 °C) was added at 1.63 L/min and sulfuric acid (1.121 g/ml) was added at a rate sufficient to maintains pH 9.6 (+/- 0.1), for an additional 180 minutes. After the additional 180 minutes elapsed, the flow of sodium silicate was stopped, and the pH was adjusted to pH 6.0 with continued flow of sulfuric acid (1.121 g/mL) at a rate of 0.82 mL/min. Once the pH was stabilized at pH 6.0, the batch was filtered, washed until a conductivity of <1500 μS, was spray dried to a target moisture of 5% and was milled to a particle size of approximately 10 μm.

Example 10C: (Inventive Example)

44 L of sodium silicate (2.65 MR, 1.193g/mL, heated to 80 °C) and 115 L of water were added to a 1200 L reactor and heated 85 °C with stirring at 80 RPM and recirculation at 80 L/min. Once 85 °C was reached, sodium silicate (2.65 MR, 1.193g/ml, heated to 80 °C) and sulfuric acid (1.121g/mL) were added simultaneously for 38 minutes at rates of 4.48 L/min and 1.51 L/min, respectively. After 38 minutes elapsed, the flow of sodium silicate and sulfuric acid were stopped, and the reaction mixture was heated to 95 °C. Once 95 °C was reached, sodium silicate (2.65 MR, 1.193g/mL, heated to 80 °C) was added at 1.73 L/min and sulfuric acid (1.121 g/ml) was added at a rate sufficient to maintains pH 9.6 (+/- 0.1), for an additional 210 minutes. After the additional 210 minutes elapsed, the flow of sodium silicate was stopped, and the pH was adjusted to pH 6.0 with continued flow of sulfuric acid (1.121 g/mL) at a rate of 0.82 mL/min. Once the pH was stabilized at pH 6.0, the batch was filtered, washed until a conductivity of <1500 μS, was spray dried to a target moisture of 5% and was milled to a particle size of approximately 10 μm.

Table 6a and 6b shows the analytical values of the Examples.

Table 6a

| Example | Total Hg intruded Volume (cc/g) | BET SA ($m^2$/g) | CTAB SA ($m^2$/g) | Oil Absorption (cc/100g) | AbC Water Absorption (cc/100g) | Median APS ($\mu$m) | Mean APS ($\mu$m) | $Na_2SO_4$ Cond ($\mu$S/cm) | Pour Density (lb/ft$^3$) | Pack Density (lb/ft$^3$) | 5% pH | 325 mesh Residue (%) | Moisture (%) | Bright ness |
|---|---|---|---|---|---|---|---|---|---|---|---|---|---|---|
| 10A | 1.50 | 20 | 30 | 84 | 102 | 6.9 | 7.4 | 850 | 16.4 | 32.9 | 6.9 | 0 | 6.1 | 96.4 |
| 10B | 1.20 | 14 | 23 | 74 | 91 | 7.3 | 8.0 | 650 | 22.3 | 41.6 | 7.1 | 0 | 4.6 | 95.4 |
| 10C | 0.95 | 15 | 21 | 64 | 82 | 6.6 | 7.2 | 600 | 25.0 | 48.0 | 7.1 | 0 | 3.4 | 94.5 |

Table 6b

| Example | Primary Particle Size Mean, SEM (nm) | Primary Particle Size Median, SEM (nm) | SEM number of particles counted | C-content (%) |
|---|---|---|---|---|
| 10A | 106 | 100 | 114 | 0 |
| 10B | 150 | 100 | 77 | 0 |
| 10C | 130 | 90 | 70 | 0 |

Example 11: Toothpaste formulation

The inventive silicas from above were incorporated into toothpaste for rheology and PCR/RDA measurements. The formulation and results (Table 7a) are shown below. The stannous compatibility and fluoride compatibility of the inventive examples are shown in Table 7b.

Table 7a

| Example | 11A | 11B | 11C | 11D | 11E | 11F | 11G | 11H |
|---|---|---|---|---|---|---|---|---|
| Glycerin, 99.7% | 11.0 | 11.0 | 11.0 | 11.0 | 11.0 | 13.2 | 13.2 | 13.2 |
| Sorbitol, 70.0% | 40.0 | 40.0 | 40.0 | 40.0 | 40.0 | 42.2 | 42.2 | 42.2 |
| Deionized Water | Q.S. | Q.S. | Q.S. | Q.S. | Q.S. | Q.S. | Q.S. | Q.S. |
| PEG-12 | 3.0 | 3.0 | 3.0 | 3.0 | 3.0 | 3.0 | 3.0 | 3.0 |
| Cekol 2000 | 1.2 | 1.2 | 1.2 | 1.2 | 1.2 | 1.2 | 1.2 | 1.2 |
| Tetrasodium Pyrophosphate | 0.5 | 0.5 | 0.5 | 0.5 | 0.5 | 0.5 | 0.5 | 0.5 |
| Sodium Saccharin | 0.2 | 0.2 | 0.2 | 0.2 | 0.2 | 0.2 | 0.2 | 0.2 |
| Sodium Fluoride | 0.243 | 0.243 | 0.243 | 0.243 | 0.243 | 0.243 | 0.243 | 0.243 |
| ZEODENT® 165 | 1.5 | 1.5 | 1.5 | 1.5 | 1.5 | 5.0 | 5.0 | 5.0 |
| ZEODENT® 103 | 20.0 | | | | | | | |
| ZEODENT® 113 | | 20.0 | | | | | | |
| Example 10A | | | 20.0 | | | 10.0. | | |
| Example 10B | | | | 20.0 | | | 10.0 | |
| Example 10C | | | | | 20.0 | | | 10.0 |
| TiO2 | 0.5 | 0.5 | 0.5 | 0.5 | 0.5 | 0.5 | 0.5 | 0.5 |
| Sodium Lauryl Sulfate | 1.2 | 1.2 | 1.2 | 1.2 | 1.2 | 1.2 | 1.2 | 1.2 |
| Flavor | 0.6 | 0.6 | 0.6 | 0.6 | 0.6 | 0.6 | 0.6 | 0.6 |
| **Total** | **100** | **100** | **100** | **100** | **100** | **100** | **100** | **100** |
| PCR | 113 | 72 | 93 | 109 | 116 | 70 | 94 | 96 |
| RDA | 220 | 109 | 114 | 154 | 200 | 78 | 119 | 149 |
| (PCR/RDA was run at the Indiana University School of Dentistry) | | | | | | | | |

Table 7b

| Example | Stannous compatibility (%) | Fluoride compatibility (%) |
|---|---|---|
| 10A | 49 | 93 |
| 10B | 60 | 94 |
| 10C | 62 | 92 |

The data in Table 7a show that the toothpaste containing the inventive silicas achieve PCR and RDA values in the normal range.

Stannous compatibility (%)

The Stannous compatibility of the above samples was determined as follows. A stock solution containing 431.11 g of 70% sorbitol, 63.62 g of de-oxygenated deionized water, 2.27 g of stannous chloride dihydrate, and 3 g of sodium gluconcate was prepared. 34 g of the stock solution was added to a 50 mL centrifuge tube containing 6 g of the silica sample to be tested. The centrifuge tube was placed on a rotating wheel at 5 RPM and aged for 1 week at 40 °C. After aging, the tube was centrifuged at 12,000 RPM for 10 minutes, and the stannous concentration in the supernatant was determined by ICP-OES (inductively coupled plasma optical emission spectrometer). The stannous compatibility was determined by expressing the stannous concentration of the sample as a percentage of the stannous concentration of a solution prepared by the same procedure, but with no silica added.

Fluoride Compatibility (%)

The Fluoride compatibility of the above samples were determined as follows. A fluoride stock solution containing 1624ppm was prepared. 30.0 g of the stock solution was added to a 50 mL centrifuge tube containing 7.0 g of the silica sample to be tested. The centrifuge tube was placed on a rotating wheel at 5 RPM (or equivalent means of agitation) and was aged for 1 hour at 60 °C. After aging, the centrifuge tube was centrifuged at 12,000 RPM for 10 minutes (or until the supernatant is clear). The Fluoride concentration in the supernatant was determined by first taking an aliquot, transferring it to a plastic vial containing a magnetic stir bar and an equal volume of TISAB II buffer. The concentration was then measured using a pre-calibrated fluoride specific ion electrode (Orion model #96-09BN or equivalent). The Fluoride compatibility was determined by expressing the Fluoride concentration of the sample as a percentage of the Fluoride concentration of a stock solution.

Relative Dentin Abrasion (RDA)

The RDA values of dentifrice compositions of Examples DC1-DC14 containing the silicas of the current invention was determined according to the method set forth by Hefferen, Journal of Dental Res., July-August 1976, 55 (4), pp. 563-573, and described in Wason U.S. Pat. Nos. 4,340,583, 4,420,312 and 4,421,527, the contents of which are incorporated herein by reference in their entirety.

Pellicle Cleaning Ratio ("PCR")

The cleaning property of dentifrice compositions was typically expressed in terms of Pellicle Cleaning Ratio ("PCR") value. The PCR test measures the ability of a dentifrice composition to remove pellicle film from a tooth under fixed brushing conditions. The PCR test is described in "In Vitro Removal of Stain with Dentifrice" G. K. Stookey, et al., J. Dental Res., 61, 12-36-9, 1982. Both PCR and RDA results vary depending upon the nature and concentration of the components of the dentifrice composition. PCR and RDA values are unit-less.

Example 12: CPC and BAC compatibility

To determine a given silicas capacity for a quaternary ammonium compound, a zeta potential titration was conducted. In the titration, a 5 wt. % suspension of the desired silica was made by taking the desired amount of dry silica and diluting to 160 g with de-ionized water. In order to get as close to the desired 5 wt. % (8g) of silica in the 160g suspension, the amount of as received silica used was adjusted to compensate for the amount of free moisture (loss on drying) and sodium sulfate present. This suspension was magnetically stirred at 500 rpm for 10 minutes to allow the silica to fully wet out then the suspensions were adjusted to a pH of ~8.5 with either 0.5M NaOH or 0.5M HCl, to help with consistency of the initial surface chemistry and a more direct comparison.

The suspensions were then titrated using 0.25ml increments of 5 wt% cetyl pyridinium Chloride (CPC) or 5 wt% Benzalkonium Chloride (BAC) and the capacity of each silica was determined by the volume of CPC or BAC required to reach a zeta potential of 0 mV. Seeing as most of the tests exhibited an artifact when approaching the 0 mV cross over, the capacity was defined as the first point at which the zeta potential became positive. The volume at this point was then used to determine the mass of CPC or BAC per gram of silica in units of mg/g.

The inventive Examples 10A, 10B and 10C show a lower CPC and BAC value and therefore an improved CPC/BAC compatibility (Table 8),

Table 8

| | CPC solution (g) | CPC-dry mass (g) | Silica-dry mass (g) | mg CPC/g silica | | BAC solution (g) | BAC-dry mass (mg) | Silica-dry mass (g) | mg BAC/g silica |
|---|---|---|---|---|---|---|---|---|---|
| ZEODENT® 113 | 5.00 | 250.0 | 4.66 | 53.66 | | 4.00 | 200.0 | 4.66 | 42.96 |
| ZEODENT® 120 | 6.75 | 337.5 | 4.62 | 73.05 | | 5.25 | 262.5 | 4.62 | 56.82 |
| ZEODENT® 103 | 4.25 | 212.5 | 4.70 | 46.40 | | 3.75 | 187.5 | 4.58 | 40.94 |
| Example 10A | 1.50 | 75.0 | 4.77 | 15.97 | | 1.50 | 75.0 | 4.70 | 15.97 |
| Example 10B | 1.00 | 50.0 | 4.77 | 10.49 | | 1.25 | 62.5 | 4.77 | 13.12 |
| Example 10C | 1.00 | 50.0 | 4.82 | 10.38 | | 1.25 | 62.5 | 4.82 | 12.98 |

Example 13: Flavor

Method: 500 mg of silica was placed in a headspace vial. 10µl of flavor (Lime Oil, Lot MKCF9356 flavor matrix) was added and the vial was allowed to equilibrate overnight. The samples were incubated at 60C for 60 minutes with gentle shaking before the headspace was sampled. 1 mL of head space was analyzed in a GC/MS with equipped with a Stabilwax column (0.25mm x 60m) with a column flow rate of 1.606 mL/min and a temperature ramp of 6°C/min over a temperature range of 40°C to 230°C (HS Sampling: 1ml of the head space was sampled into a gas tight syringe at 65C). Peak areas were standardized relative to the peak intensity for ZEODENT® 113.

The inventive Examples 10A, 10B and 10C show higher values and therefore an improved flavor compatibility (Table 9),

Table 9

| | α-pinene | β-pinene | β-myrcene | α-terpinene | limonene | eucalyptol | γ-terpinene | o-cymene | terpinolene | dehydro-p-cymene | Sum of ratio |
|---|---|---|---|---|---|---|---|---|---|---|---|
| ZEODENT® 113 | 1 | 1 | 1 | 1 | 1 | 1 | 1 | 1 | 1 | 1 | 10.00 |
| ZEODENT® 120 | 0.87 | 0.79 | 0.76 | 0.90 | 0.85 | 0.81 | 0.83 | 0.76 | 0.80 | 0.00 | 7.37 |
| ZEODENT® 103 | 1.02 | 1.06 | 1.27 | 1.34 | 1.09 | 1.24 | 1.13 | 1.06 | 1.16 | 1.25 | 11.62 |
| Example 10A | 1.35 | 1.46 | 2.04 | 1.51 | 1.53 | 2.38 | 1.64 | 2.79 | 1.71 | 2.38 | 18.80 |
| Example 10B | 1.54 | 1.68 | 2.50 | 2.01 | 1.79 | 4.55 | 1.85 | 4.60 | 1.98 | 3.40 | 25.91 |
| Example 10C | 1.49 | 1.50 | 2.33 | 2.02 | 1.70 | 3.69 | 1.89 | 3.50 | 2.00 | 3.06 | 23.18 |

**Claims**

1. A precipitated silica **characterized by** a primary particle size mean of greater than 80 nm, a BET surface area of 10-40 $m^2$/g, a total mercury intruded volume of 0.75-2.00 cc/g, and an oil absorption of 60-120 cc/100g.

2. The precipitated silica according to claim 1, wherein the precipitated silica has a CTAB surface area of 10-35 $m^2$/g, preferably 20-30 $m^2$/g.

3. The precipitated silica according to claim 1 or 2, wherein the precipitated silica has a pack density of 0.40-0.80 $g/cm^3$, preferably 0.48-0.75 $g/cm^3$.

4. The precipitated silica according to any of claims 1-3, wherein the precipitated silica has an Einlehner value of less than 18 mg lost/100k revolutions, preferably 3 to 18 mg lost/100k revolutions.

5. The precipitated silica according to any of claims 1-4, wherein the precipitated silica has a C-content of lower than 3%, preferably lower than 1%, more preferably 0% to 0.5 %.

6. The precipitated silica of claim 1, wherein the precipitated silica has a primary particle size mean of greater than 90 nm, a BET surface area of 10-26 $m^2$/g, a total mercury intruded volume of 0.75-2.00 cc/g and an oil absorption of 60-120 cc/100g.

7. The precipitated silica of claim 1, wherein the precipitated silica has a primary particle size mean of greater than 80 nm, a BET surface area of 10-30 $m^2$/g, a total mercury intruded volume of 0.80-1.80 cc/g and an oil absorption of 70-110 cc/100g.

8. The precipitated silica of claim 1, wherein the precipitated silica has a primary particle size mean of greater than 90 nm, a BET surface area of 10-26 $m^2$/g, a total mercury intruded volume of 0.80-1.80 cc/g and an oil absorption of 70-110 cc/100g.

9. The precipitated silica of claim 1, wherein the precipitated silica has a primary particle size mean of greater than 100 nm, a BET surface area of 10-23 $m^2$/g, a total mercury intruded volume of 0.75-2.00 cc/g and an oil absorption of 60-120 cc/100g.

10. The precipitated silica of claim 1, wherein the precipitated silica has a primary particle size mean of greater than 80 nm, a BET surface area of 10-30 $m^2$/g, a total mercury intruded volume of 0.85-1.65 cc/g and an oil absorption of 60-100 cc/100g.

11. The precipitated silica of claim 1, wherein the precipitated silica has a primary particle size mean of greater than 100 nm, a BET surface area of 10-23 $m^2$/g, a total mercury intruded volume of 0.85-1.65 cc/g and an oil absorption of 60-100 cc/100g.

12. A process for the production of precipitated silica according to claim 1 comprises

    (a) continuously feeding an acid and an alkali metal silicate or alkaline earth metal into a liquid medium at a silicate addition rate V1 and a temperature 70-96°C with stirring to form the silica particles, , wherein the liquid medium in step is alkali metal silicate or alkaline earth metal silicate and water,
    (b) stopping the feed of alkali metal silicate or alkaline earth metal silicate and acid and then increasing the temperature to 90 to 100°C, preferably 94 to 96 °C, with stirring,
    (c) adding an alkali metal silicate or alkaline earth metal silicate and an acid with stirring, wherein the silicate addition rate is 1 to 40 %, preferably 1 to 30 %, more preferably 2 to 10 %, still preferably 3 to 5 %, of the silicate addition rate V1 and the pH value of 9.0 to 10.0, preferably 9.5 to 9.9, more preferably 9.6 to 9.8, is kept constant during the addition of the alkali metal silicate or alkaline earth metal silicate, by adjusting the acid rate, wherein the period of time for this step is 100 to 500 minutes,
    (d) stopping the addition of alkali metal silicate or alkaline earth metal silicate and adding acid with stirring until the pH of 5.0 to 7.0, preferably 5.5 to 6.5, is reached.

13. The process for the production of precipitated silica of claim 12, wherein the silica of step (d) is filtered (e) and dried in a spray dryer (f) and wherein the silica of step (f) is milled.

14. Use of precipitated silica of claim 1 in cosmetics, anti-caking free/flow, food, carrier applications, dentifrice and mouthwash.

15. Dentifrice composition comprising the precipitated silica of any claims 1- 11.

**Patentansprüche**

1. Fällungskieselsäure, **gekennzeichnet durch** einen Primärteilchengrößen-Mittelwert von mehr als 80 nm, eine BET-Oberfläche von 10-40 m$^2$/g, ein Gesamtquecksilberintrusionsvolumen von 0,75-2,00 cm$^3$/g und eine Ölabsorption von 60-120 cm$^3$/100 g.

2. Fällungskieselsäure nach Anspruch 1, wobei die Fällungskieselsäure eine CTAB-Oberfläche von 10-35 m$^2$/g, vorzugsweise 20-30 m$^2$/g, aufweist.

3. Fällungskieselsäure nach Anspruch 1 oder 2, wobei die Fällungskieselsäure eine Packungsdichte von 0,40-0,80 g/cm$^3$, vorzugsweise 0,48-0,75 g/cm$^3$, aufweist.

4. Fällungskieselsäure nach einem der Ansprüche 1-3, wobei die Fällungskieselsäure einen Einlehner-Wert von weniger als 18 mg Verlust/100.000 Umdrehungen, vorzugsweise 3 bis 18 mg Verlust/100.000 Umdrehungen, aufweist.

5. Fällungskieselsäure nach einem der Ansprüche 1-4, wobei die Fällungskieselsäure einen C-Gehalt von weniger als 3 %, vorzugsweise weniger als 1 %, weiter bevorzugt 0 % bis 0,5 %, aufweist.

6. Fällungskieselsäure nach Anspruch 1, wobei die Fällungskieselsäure einen Primärteilchengrößen-Mittelwert von mehr als 90 nm, eine BET-Oberfläche von 10-26 m$^2$/g, ein Gesamtquecksilberintrusionsvolumen von 0,75-2,00 cm$^3$/g und eine Ölabsorption von 60-120 cm$^3$/100 g aufweist.

7. Fällungskieselsäure nach Anspruch 1, wobei die Fällungskieselsäure einen Primärteilchengrößen-Mittelwert von mehr als 80 nm, eine BET-Oberfläche von 10-30 m$^2$/g, ein Gesamtquecksilberintrusionsvolumen von 0,80-1,80 cm$^3$/g und eine Ölabsorption von 70-110 cm$^3$/100 g aufweist.

8. Fällungskieselsäure nach Anspruch 1, wobei die Fällungskieselsäure einen Primärteilchengrößen-Mittelwert von mehr als 90 nm, eine BET-Oberfläche von 10-26 m$^2$/g, ein Gesamtquecksilberintrusionsvolumen von 0,80-1,80 cm$^3$/g und eine Ölabsorption von 70-110 cm$^3$/100 g aufweist.

9. Fällungskieselsäure nach Anspruch 1, wobei die Fällungskieselsäure einen Primärteilchengrößen-Mittelwert von mehr als 100 nm, eine BET-Oberfläche von 10-23 m$^2$/g, ein Gesamtquecksilberintrusionsvolumen von 0,75-2,00 cm$^3$/g und eine Ölabsorption von 60-120 cm$^3$/100 g aufweist.

10. Fällungskieselsäure nach Anspruch 1, wobei die Fällungskieselsäure einen Primärteilchengrößen-Mittelwert von mehr als 80 nm, eine BET-Oberfläche von 10-30 m$^2$/g, ein Gesamtquecksilberintrusionsvolumen von 0,85-1,65 cm$^3$/g und eine Ölabsorption von 60-100 cm$^3$/100 g aufweist.

11. Fällungskieselsäure nach Anspruch 1, wobei die Fällungskieselsäure einen Primärteilchengrößen-Mittelwert von mehr als 100 nm, eine BET-Oberfläche von 10-23 m$^2$/g, ein Gesamtquecksilberintrusionsvolumen von 0,85-1,65 cm$^3$/g und eine Ölabsorption von 60-100 cm$^3$/100 g aufweist.

12. Verfahren zur Herstellung von Fällungskieselsäure gemäß Anspruch 1, umfassend

(a) kontinuierliches Zuführen einer Säure und eines Alkalimetallsilikats oder Erdalkalimetalls in ein flüssiges Medium bei einer Silikatzugaberate V1 und einer Temperatur 70-96 °C unter Rühren unter Bildung der Kieselsäureteilchen, wobei das flüssige Medium in Schritt Alkalimetallsilikat oder Erdalkalimetallsilikat und Wasser ist,
(b) Stoppen der Zufuhr von Alkalimetallsilikat oder Erdalkalimetallsilikat und Säure und anschließendes Erhöhen der Temperatur auf 90 bis 100 °C, vorzugsweise 94 bis 96 °C, unter Rühren,
(c) Zugeben eines Alkalimetallsilikats oder Erdalkalimetallsilikats und einer Säure unter Rühren, wobei die Silikatzugaberate 1 bis 40 %, vorzugsweise 1 bis 30 %, weiter bevorzugt 2 bis 10 %, noch weiter bevorzugt 3 bis 5 %, der Silikatzugaberate V1 beträgt und der pH-Wert von 9,0 bis 10,0, vorzugsweise 9,5 bis 9,9, weiter bevorzugt 9,6 bis 9,8, während der Zugabe des Alkalimetallsilikats oder Erdalkalimetallsilikats durch Einstellen der Säurerate konstant gehalten wird, wobei die Zeitdauer für diesen Schritt 100 bis 500 Minuten beträgt,

d) Stoppen der Zugabe von Alkalimetallsilikat oder Erdalkalimetallsilikat und Zugeben von Säure unter Rühren bis zum Erreichen des pH-Werts von 5,0 bis 7,0, vorzugsweise 5,5 bis 6,5.

13. Verfahren zur Herstellung von Fällungskieselsäure nach Anspruch 12, wobei die Kieselsäure aus Schritt (d) filtriert wird (e) und in einem Sprühtrockner getrocknet wird (f) und wobei die Kieselsäure aus Schritt (f) gemahlen wird.

14. Verwendung von Fällungskieselsäure nach Anspruch 1 in Kosmetika, Antiback/Fließ, Lebensmitteln, Trägeranwendungen, Zahnreinigungsmittel und Mundwasser.

15. Zahnreinigungsmittelzusammensetzung, umfassend die Fällungskieselsäure nach einem der Ansprüche 1-11.

**Revendications**

1. Silice précipitée **caractérisée par** une moyenne de taille de particules primaires supérieure à 80 nm, une surface BET de 10-40 $m^2$/g, un volume total de pénétration de mercure de 0,75 à 2,00 $cm^3$/g et une absorption d'huile de 60-120 $cm^3$/100 g.

2. Silice précipitée selon la revendication 1, dans laquelle la silice précipitée a une surface CTAB de 10 à 35 $m^2$/g, de préférence 20 à 30 $m^2$/g.

3. Silice précipitée selon la revendication 1 ou 2, dans laquelle la silice précipitée a une densité compactée de 0,40 à 0,80 g/$cm^3$, de préférence de 0,48 à 0,75 g/$cm^3$.

4. Silice précipitée selon l'une quelconque des revendications 1 à 3, dans laquelle la silice précipitée a une valeur Einlehner inférieure à 18 mg perdus/100 000 tours, de préférence 3 à 18 mg perdus/100 000 tours.

5. Silice précipitée selon l'une quelconque des revendications 1 à 4, dans laquelle la silice précipitée a une teneur en C inférieure à 3 %, de préférence inférieure à 1 %, plus préférablement de 0 % à 0,5 %.

6. Silice précipitée selon la revendication 1, dans laquelle la silice précipitée a une taille moyenne de particules primaires supérieure à 90 nm, une surface BET de 10-26 $m^2$/g, un volume total de pénétration de mercure de 0,75 à 2,00 $cm^3$/g et une absorption d'huile de 60-120 $cm^3$/100 g.

7. Silice précipitée selon la revendication 1, dans laquelle la silice précipitée a une taille moyenne de particules primaires supérieure à 80 nm, une surface BET de 10-30 $m^2$/g, un volume total de pénétration de mercure de 0,80 à 1,80 $cm^3$/g et une absorption d'huile de 70-110 $cm^3$/100 g.

8. Silice précipitée selon la revendication 1, dans laquelle la silice précipitée a une taille moyenne de particules primaires supérieure à 90 nm, une surface BET de 10-26 $m^2$/g, un volume total de pénétration de mercure de 0,80 à 1,80 $cm^3$/g et une absorption d'huile de 70-110 $cm^3$/100 g.

9. Silice précipitée selon la revendication 1, dans laquelle la silice précipitée a une taille moyenne de particules primaires supérieure à 100 nm, une surface BET de 10-23 $m^2$/g, un volume total de pénétration de mercure de 0,75 à 2,00 $cm^3$/g et une absorption d'huile de 60-120 $cm^3$/100 g.

10. Silice précipitée selon la revendication 1, dans laquelle la silice précipitée a une taille moyenne de particules primaires supérieure à 80 nm, une surface BET de 10-30 $m^2$/g, un volume total de pénétration de mercure de 0,85 à 1,65 $cm^3$/g et une absorption d'huile de 60-100 $cm^3$/100 g.

11. Silice précipitée selon la revendication 1, dans laquelle la silice précipitée a une taille moyenne de particules primaires supérieure à 100 nm, une surface BET de 10-23 $m^2$/g, un volume total de pénétration de mercure de 0,85 à 1,65 $cm^3$/g et une absorption d'huile de 60-100 $cm^3$/100 g.

**12.** Procédé pour la production de silice précipitée selon la revendication 1, comprenant

(a) l'alimentation de manière continue d'un acide et d'un silicate de métal alcalin ou d'un métal alcalino-terreux dans un milieu liquide à une vitesse d'ajout de silicate V1 et à une température de 70-96 °C sous agitation pour former les particules de silice, dans lequel le milieu liquide dans l'étape est un silicate de métal alcalin ou un silicate de métal alcalino-terreux et de l'eau,

(b) l'arrêt de l'alimentation en silicate de métal alcalin ou en silicate de métal alcalino-terreux et en acide, et puis l'augmentation de la température jusqu'à 90 à 100 °C, de préférence 94 à 96 °C, sous agitation,

(c) l'ajout d'un silicate de métal alcalin ou d'un silicate de métal alcalino-terreux et d'un acide sous agitation, la vitesse d'ajout de silicate étant de 1 à 40 %, de préférence de 1 à 30 %, plus préférablement de 2 à 10 %, encore plus préférablement de 3 à 5 %, du taux d'ajout de silicate V1 et la valeur de pH de 9,0 à 10,0, de préférence de 9,5 à 9,9, plus préférablement de 9,6 à 9,8, étant maintenue constante pendant l'ajout du silicate de métal alcalin ou du silicate de métal alcalino-terreux, par ajustement de la vitesse d'acide, la période de temps pour cette étape étant de 100 à 500 minutes,

(d) l'arrêt de l'ajout de silicate de métal alcalin ou de silicate de métal alcalino-terreux et l'ajout d'acide sous agitation jusqu'à atteindre le pH de 5,0 à 7,0, de préférence de 5,5 à 6,5.

**13.** Procédé pour la production de silice précipitée selon la revendication 12, dans lequel la silice de l'étape (d) est filtrée (e) et séchée dans un séchoir par pulvérisation (f) et dans lequel la silice de l'étape (f) est broyée.

**14.** Utilisation de la silice précipitée selon la revendication 1 dans les produits cosmétiques, l'anti-agglomération libre/fluide, les aliments, les applications de support, les dentifrices et les bains de bouche.

**15.** Composition de dentifrice comprenant la silice précipitée selon l'une quelconque des revendications 1 à 11.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8597425 B
- US 6946119 B
- US 7255852 B
- US 7438895 B
- US 10328002 B
- WO 2018114280 A
- US 20190374448 A
- WO 2019238777 A
- US 2012216719 A1
- US 4340583 A, Wason
- US 4420312 A
- US 4421527 A

**Non-patent literature cited in the description**

- *J. Soc Cosmet. Chem*, August 1978, vol. 29, 497-521
- *Physiological Behavior of highly dispersed Oxides of Silicon, Aluminum and Titanium*, 1978, 26-27
- **BRUNAUR et al.** *J. Am. Chem. Soc.*, 1938, vol. 60, 309
- **HEFFEREN**. *Journal of Dental Res.*, July 1976, vol. 55 (4), 563-573
- **G. K. STOOKEY et al.** In Vitro Removal of Stain with Dentifrice. *J. Dental Res.*, 1982, vol. 61, 12-36, 9